(19) Europäisches Patentamt
European Patent Office
Office européen des brevets

(11) Veröffentlichungsnummer : **0 142 657**
**B1**

(12) # EUROPÄISCHE PATENTSCHRIFT

(45) Veröffentlichungstag der Patentschrift : **30.12.86**

(51) Int. Cl.[4] : **C 07 C 33/035**, C 07 C 29/56

(21) Anmeldenummer : **84110843.4**

(22) Anmeldetag : **12.09.84**

(54) **Verfahren zur Herstellung von 1-Buten-3,4-diol.**

(30) Priorität : **24.09.83 DE 3334589**

(43) Veröffentlichungstag der Anmeldung : **29.05.85 Patentblatt 85/22**

(45) Bekanntmachung des Hinweises auf die Patenterteilung : **30.12.86 Patentblatt 86/52**

(84) Benannte Vertragsstaaten : **BE DE FR GB IT NL**

(56) Entgegenhaltungen :
**DE-A- 2 414 341**
**DE-A- 2 605 241**
**DE-A- 3 022 288**
**DE-C- 961 353**
**US-A- 4 209 651**
**THE JOURNAL OF THE AMERICAN CHEMICAL SOCIETY, Band 80, 1958, EASTON, PA., C.T. HANDY, H.S. ROTHROCK, "Polymeric Peroxide of 1,3-Butadiene", Seiten 5306-5308**
**Die Akte enthält technische Angaben, die nach dem Eingang der Anmeldung eingereicht wurden und die nicht in dieser Patentschrift enthalten sind.**

(73) Patentinhaber : **BAYER AG**
**Konzernverwaltung RP Patentabteilung**
**D-5090 Leverkusen 1 Bayerwerk (DE)**

(72) Erfinder : **Schalenbach, Rolf, Dr.**
**In der Kreuzau 5a**
**D-5000 Köln 91 (DE)**
Erfinder : **Waldmann, Helmut, Dr.**
**Henry T.v.Böttinger Strasse 15**
**D-5090 Leverkusen 1 (DE)**

**EP 0 142 657 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft ein neues Verfahren zur Herstellung von 1-Buten-3,4-diol.

Ungesättigte $C_4$-Diole sind als Zwischenprodukte für Wirkstoffe und als Polymergrundstoffe von wirtschaftlichem Interesse (siehe Ullmanns Enzyclopädie der technischen Chemie, 4. Auflage, Band 9, Seite 19 ; Kirk-Othmer, Encyclopedia of Chemical Technology, 3rd edition, Vol. 1, Seite 256 (1978)).

Die Herstellung des 1-Buten-3,4-diols erfolgt bisher beispielsweise durch die Hydrierung von polymeren Peroxiden des Butadiens (siehe US-PS 4 209 651 und US-PS 2 898 377). Nachteilig bei diesem Verfahren sind die geringen Ausbeuten, in denen polymere Peroxide bei der Oxidation von Butadien erhältlich sind und die Bildung eines Gemisches von 1,2- und 1,4-Addukt (siehe US-PS 3 023 249 ; C. T. Handy, H. S. Rothrock, J. Am. Chem. Soc. *80*, 5306 (1958)).

Ein weiterer Zugang zu 1-Buten-3,4-diol ist die Hydrolyse von Vinyloxiran (siehe JP-OS 79/79214 ; W. F. Whitmore, J. Am. Chem. Soc. 71, 2427 (1949)). Diese Verfahrensweise hat den Nachteil, daß Vinyloxiran derzeitig in technischen Mengen nicht verfügbar ist, sondern erst aus Butadien hergestellt werden müßte.

Weiterhin kann 1-Buten-3,4-diol durch Hydrolyse von 3,4-Diacetoxybuten gewonnen werden, das als Nebenprodukt bei Acetoxylierung von Butadien zum 1,4-Diacetoxybuten-2 anfällt (siehe DE-OS 3 022 288). Die Durchführung dieses Verfahrens wird dadurch erschwert, daß 3,4-Diacetoxybuten-2 nur in geringen Mengen anfällt und die Trennung der isomeren Diacetate schwierig ist.

Das zum 1-Buten-3,4-diol strukturisomere 2-Buten-1,4-diol ist in technischem Maßstab durch Hydrierung von 2-Butin-1,4-diol verfügbar (siehe US-PS 4 213 000 ; DE-OS 2 605 241 ; DE-OS 2 431 929), das bekanntermaßen durch Umsetzung von Acetylen und Formaldehyd hergestellt werden kann (siehe US-PS 3 560 576 ; US-PS 3 920 759 ; DE-OS 2 314 693). Die Umlagerung von 2-Buten-1,4-diol zu 1-Buten-3,4-diol zu 1-Buten-3,4-diol würde es ermöglichen, ausgehend vom großtechnisch verfügbaren 2-Butin-1,4-diol auf einfachem Wege und ohne die Bildung und Isolierung von Zwischenprodukten zum 1-Buten-3,4-diol zu gelangen.

Es ist zwar grundsätzlich bekannt, daß man an Verbindungen mit Allylalkoholstruktur Isomerisierungsreaktionen durchführen kann (siehe C. Ferri, Reaktionen der organischen Chemie, Georg Thieme Verlag, 1978, Seite 243). Die Umlagerungen von Allylalkoholen verlaufen jedoch häufig nicht in der erwünschten Weise. Werden solche Reaktionen in der Gasphase durchgeführt, so isomerisieren die ungesättigten Alkohole zu den entsprechenden Aldehyden und Ketonen (siehe M. Kraus, Coll. Czech. Chem. Commun. *37*, 460 (1972) ; G. Eadon, M. Y. Shiekh, J. Am. Chem. Soc. *96*, 2288 (1974)).

In der flüssigen Phase können Allylalkohole beim Behandeln mit Säure unter Erhalt der Hydroxylfunktion umgelagert werden (siehe Braude, Quart. Rev. *4*, 407 (1950)). Aus der US-PS 2 373 956 ist bekannt, daß 3-Penten-1,2-diol beim Erhitzen in verdünnter Schwefelsäure in 2-Penten-1,4-diol überführt wird.

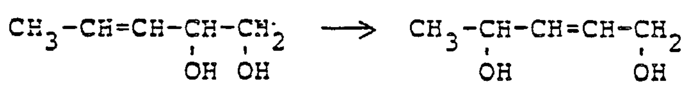

Dies ist der umgekehrte Vorgang dessen, was bei der Isomerisierung von 2-Buten-1,4-diol zu 1-Buten-3,4-diol gewünscht wird.

In der DE-PS 961 353 wird die Reaktion von 2-Buten-1,4-diol mit $CuCl_2$ in Abwesenheit von Säuren beschrieben. Beim Erhitzen von 2-Buten-1,4-diol unter Rückfluß in Gegenwart von $CuCl_2$ wird Divinyldioxan-1,4 gebildet.

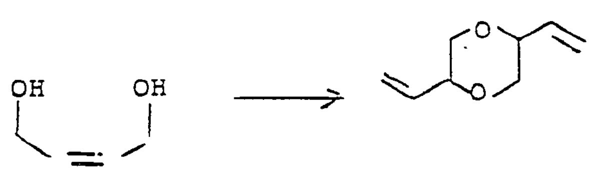

Die bisher bekannt gewordenen Verfahren zur Herstellung von 1-Buten-3,4-diol sind demnach für eine technische Herstellung nicht geeignet und nach dem einschlägigen Stand der Technik war nicht zu erwarten, daß 2-Buten-1,4-diol in 1-Buten-3,4-diol umgelagert werden kann.

Es wurde nun ein Verfahren zur Herstellung von 1-Buten-3,4-diol gefunden, das dadurch gekennzeichnet ist, daß man 2-Buten-1,4-diol in Gegenwart katalytisch wirksamer Substanzen und unter Hinzufügen einer organischen oder anorganischen Säure thermisch behandelt.

Prinzipiell ist jedes 2-Buten-1,4-diol zur Durchführung des erfindungsgemäßen Verfahrens geeignet, beispielsweise ein technisches Produkt, das durch Hydrierung von 2-Butin-1,4-diol hergestellt wurde.

Katalytisch wirksame Substanzen für das erfindungsgemäße Verfahren sind beispielsweise die Elemente der 3. und 4. Hauptgruppe des Periodensystems der Elemente nach Mendelejew und die Übergangsmetalle, sowie Verbindungen dieser Elemente und Verbindungen von Übergangsmetallen. Bevorzugt sind Bor, Aluminium, Zinn, Blei, Kupfer, Zink, Eisen, Silber, Cadmium, Ruthenium, Palladium,

Gold und Quecksilber und deren Verbindungen. Besonders bevorzugt sind die Elemente der 1. und 2. Nebengruppe des Periodensystems der Elemente nach Mendelejew und ihre Verbindungen, ganz besonders Kupfer und seine Verbindungen.

Geeignete Verbindungen der genannten Elemente und Übergangsmetalle sind beispielsweise solche, die Sauerstoff, Stickstoff, Schwefel, Phosphor, Halogen und/oder Kohlenstoff enthalten, beispielsweise Oxide, Nitrate, Sulfate, Phosphate, Fluoride, Chloride, Bromide, Jodide, Perchlorate, Acetate, Trifluoracetate und ähnliche.

Bevorzugte Einzelverbindungen sind z. B. $BF_3$, $AlCl_3$, $SnCl_2$, $SnCl_4$, $Pb(acetat)_2$, $CuSO_4$, $Cu(NO_3)_2$, $Cu(acetat)_2$, $CuCl_2$, CuCl, CuJ, CuBr, $ZnCl_2$, $HgCl_2$, $AgClO_4$, $AgO_2CCF_3$, $FeCl_2$, $FeCl_3$, $CdBr_2$, $Pd(acetat)_2$, $PdCl_2$, $AuCl_3$ und $RuCl_3$.

Besonders bevorzugt sind Verbindungen des Kupfers, insbesondere die des einwertigen Kupfers wie CuCl, CuBr und CuJ, ganz besonders aber CuCl.

Für das erfindungsgemäße Verfahren ist es nicht entscheidend, ob die genannten Elemente und Verbindungen als solche eingesetzt werden, sich erst unter den Verfahrensbedingungen bilden oder während des Verfahrens umgewandelt werden.

Die Menge der katalytisch wirksamen Substanzen kann im erfindungsgemäßen Verfahren in weiten Grenzen variiert werden. Bezogen auf das eingesetzte 2-Buten-1,4-diol können sie beispielsweise in Mengen zwischen 0,1 und 25 Gew.-%, bevorzugt in Mengen zwischen 1 und 10 Gew.-%, besonders bevorzugt in Mengen zwischen 2 und 5 Gew.-%, eingesetzt werden. Diese Grenzen sind Richtwerte, die sowohl unter- als auch überschritten werden können.

In die erfindungsgemäße Isomerisierung werden Säuren eingesetzt. Grundsätzlich geeignet sind sowohl organische als auch anorganische Säuren, beispielsweise Ameisensäure, Essigsäure, Trifluoressigsäure, Phosphorsäure, Salzsäure, Schwefelsäure und Perchlorsäure, aber auch saure Ionenaustauscher. Bevorzugt sind Salzsäure und Schwefelsäure, insbesondere Salzsäure.

Die Menge der zuzusetzenden Säure kann in weiten Grenzen variiert werden. So kann die Menge, bezogen auf eingesetztes 2-Buten-1,4-diol, beispielsweise 0,5-40 Gew.-% betragen. Bevorzugt ist hier eine Menge von 5-30 Gew.-%, besonders bevorzugt eine solche von 10-25 Gew.-%. Die angegebenen Grenzen können auch unter- oder überschritten werden.

Es kann vorteilhaft sein, das erfindungsgemäße Verfahren in Gegenwart eines Lösungsmittels durchzuführen. Als Lösungsmittel kommen beispielsweise Alkohole, Ether, Polyether, Ester, Säuren und Wasser in Frage. Es ist vorteilhaft, solche Lösungsmittel anzuwenden, deren Siedepunkt im Bereich der zur thermischen Behandlung notwendigen Temperatur liegt. Bevorzugte Lösungsmittel sind Ethanol, Wasser, Dimethoxyethan und Essigsäure. Besonders bevorzugt ist Wasser. Die Menge des Lösungsmittels kann in großem Umfang variiert werden. So können Lösungsmittel, bezogen auf eingesetztes 2-Buten-1,4-diol, beispielsweise in der 0,1-20-fachen Gewichtsmenge eingesetzt werden. Bevorzugt ist hier die 1-12-fache, besonders bevorzugt die 2-10-fache Menge.

Als thermische Behandlung im Sinne der vorliegenden Erfindung ist ein inniger Kontakt des Reaktionsgemisches bei ausreichender Temperatur zu verstehen. Ausreichende Temperaturen für das erfindungsgemäße Verfahren sind im allgemeinen schon solche von 50-60 °C. Um eine höhere Reaktionsgeschwindigkeit zu erreichen, ist es vorteilhaft, bei höheren Temperaturen, z. B. bei 60-150 °C zu arbeiten. Man kann auch höhere Temperaturen anwenden, beispielsweise solche von bis zu 190 °C und darüber. Besonders bevorzugt sind Temperaturen von 80-120 °C.

Ein inniger Kontakt des Reaktionsgemisches kann beispielsweise durch Rühren erreicht werden.

Je nachdem, ob man das erfindungsgemäße Verfahren bei höherer oder tieferer Temperatur durchgeführt wird, sind zur Erzielung guter Ausbeuten verschieden lange Verweilzeiten nötig. Bei höherer Temperatur sind zur Erzielung guter Ausbeuten im allgemeinen kürzere Verweilzeiten möglich als bei tierferer Temperatur. Im allgemeinen liegen die Verweilzeiten im Bereich von 1-15 Stunden.

Der Druck ist für die Durchführung des erfindungsgemäßen Verfahrens von untergeordneter Bedeutung. Normalerweise wird man bei Atmosphärendruck arbeiten. Man kann aber auch bei erhöhtem oder erniedrigtem Druck arbeiten.

Die Isolierung des 1-Buten-3,4-diols aus dem nach Durchführung des erfindungsgemäßen Verfahrens vorliegenden Reaktionsgemisch kann durch Destillation erfolgen, gegebenenfalls durch Destillation unter vermindertem Druck. Um Zersetzungen während der Destillation zu vermeiden, ist es vorteilhaft, das Reaktionsgemisch vor der Destillation zu neutralisieren und dabei gegebenenfalls ausfallende Feststoffe abzufiltrieren.

Die Durchführung des erfindungsgemäßen Verfahrens kann kontinuierlich und diskontinuierlich erfolgen. Geeignete Reaktionsgefäße sind z. B. Rührreaktoren oder Kaskaden von Rührreaktoren. Als Werkstoffe für die Reaktionsgefäße kommen beispielsweise Edelstähle, Glas, Emaille, Tantal und Titan in Frage.

Bei einer bevorzugten Durchführungsform des erfindungsgemäßen Verfahrens wird 2-Buten-1,4-diol, das aus der Hydrierung von 2-Butin-1,4-diol erhalten wurde, mit einem Lösungsmittel, einer Säure und einer katalytisch wirksamen Substanz in einem Rührkessel zusammengebracht. Dann wird die Reaktionsmischung auf die gewünschte Temperatur erwärmt und gerührt. Nach der Neutralisation und gegebenenfalls nach der Abtrennung von ausgefallenen Feststoffen wird das 1-Buten-3,4-diol destillativ isoliert.

Anhand der folgenden Beispiele wird das erfindungsgemäße Verfahren weiter verdeutlicht.

Beispiel 1

50 g 2-Buten-1,4-diol wurden in 250 ml Wasser gelöst und nach Zugabe von 10 ml 37 %-iger wäßriger Salzsäure und von 2 g CuCl 2 Stunden lang auf 90 °C (Innentemperatur) erhitzt und dabei gerührt. Die gaschromatographische Analyse des Rohproduktes ergab einen Gehalt von 8,5 Gew.-% 1-Buten-3,4-diol und 6,3 Gew.-% 2-Buten-1,4-diol. Das entspricht einem Umsatz von 2-Buten-1,4-diol von 61 % und einer Selektivität für 1-Buten-3,4-diol von 85 %.

Beispiel 2

100 g 2-Buten-1,4-diol wurden in 600 ml Wasser gelöst und nach Zugabe von 5 ml konz. wäßriger Salzsäure und 1 g CuCl auf 100 °C erwärmt. Nach 15-stündigem Rühren enthielt das Reaktionsgemisch 48 g 1-Buten-3,4-diol. Das eingesetzte 2-Buten-1,4-diol wurde zu 74 % umgesetzt.

Beispiel 3

1 900 g 2-Buten-1,4-diol wurden in einem Rührkessel mit 9 500 ml Wasser versetzt. Nach Zugabe von 380 ml wäßriger Salzsäure (30 Gew.-%ig) und 76 g CuCl wurde die Reaktionsmischung zum Sieden erhitzt. Nach 2-stündigem Rühren bei dieser Temperatur enthielt das Reaktionsgemisch 790 g 1-Buten-3,4-diol. Zur Isolierung des 1-Buten-3,4-diols wurde das Reaktionsgemisch mit Natronlauge neutralisiert und durch Filtration von den ausgefallenen Feststoffen befreit. Die destillative Aufarbeitung des verbliebenen Gemisches bei vermindertem Druck (1 mbar) lieferte 776 g 1-Buten-3,4-diol, entsprechend einer Menge von 98 % des im Rohprodukt vorhandenen 1-Buten-3,4-diols.

Beispiel 4

55 g 2-Buten-1,4-diol wurden in 250 ml Wasser gelöst und nach Zugabe von 2 g $HgCl_2$ und 10 ml wässriger Salzsäure (37 %-ig) unter Rühren bei mildem Rückfluß erhitzt.

Nach 11 Stunden waren 58 % des eingesetzten 2-Buten-1,4-diols umgesetzt. Die Selektivität für 1-Buten-3,4-diol betrug 82 %.

## Patentansprüche

1. Verfahren zur Herstellung von 1-Buten-3,4-diol, dadurch gekennzeichnet, daß man 2-Buten-1,4-diol in Gegenwart katalytisch wirksamer Substanzen und unter Hinzufügen einer organischen oder anorganischen Säure thermisch behandelt.

2. Verfahren nach Anspruch 1, dadurch gekennzeichnet, daß man das Verfahren in Gegenwart von Elementen der dritten und vierten Hauptgruppe des Periodensystems der Elemente nach Mendelejew, von Übergangsmetallen oder von Verbindungen dieser Elemente oder von Verbindungen der Übergangsmetalle durchführt.

3. Verfahren nach Ansprüchen 1 und 2, dadurch gekennzeichnet, daß man das Verfahren in Gegenwart von Elementen der ersten oder zweiten Nebengruppe des Periodensystems der Elemente nach Mendelejew oder deren Verbindungen durchführt.

4. Verfahren nach Ansprüchen 1 bis 3, dadurch gekennzeichnet, daß man das Verfahren in Gegenwart von Kupfer oder Kupferverbindungen durchführt.

5. Verfahren nach Ansprüchen 1 bis 4, dadurch gekennzeichnet, daß man eine Säure aus der Gruppe Ameisensäure, Essigsäure, Trifluoressigsäure, Phosphorsäure, Salzsäure, Schwefelsäure, Perchlorsäure und sauren Ionenaustauschern zufügt.

6. Verfahren nach Ansprüchen 1 bis 5, dadurch gekennzeichnet, daß man das Verfahren bei 50 bis 190 °C durchführt.

7. Verfahren nach Ansprüchen 1 bis 6, dadurch gekennzeichnet, daß man das Verfahren in Gegenwart eines Lösungsmittels durchführt.

8. Verfahren nach Ansprüchen 1 bis 7, dadurch gekennzeichnet, daß man, bezogen auf 2-Buten-1,4-diol, 0,1 bis 25 Gew.-% katalytisch wirksame Substanzen einsetzt.

9. Verfahren nach Ansprüchen 1 bis 8, dadurch gekennzeichnet, daß man, bezogen auf 2-Buten-1,4-diol, 0,5 bis 40 Gew.-% Säure zusetzt.

## Claims

1. Process for the preparation of 1-butene-3,4-diol, characterised in that 2-butene-1,4-diol is heat treated in the presence of catalytically active substances and with the addition of an organic or inorganic

acid.

2. Process according to Claim 1, characterised in that the process is carried out in the presence of elements of the third and fourth main groups of the Mendeleev periodic table of the elements, of transition metals or of compounds of these elements or of compounds of the transition metals.

3. Process according to Claims 1 and 2, characterised in that the process is carried out in the presence of elements of the first or second sub-group of the Mendeleev periodic table of the elements or of their compounds.

4. Process according to Claims 1 to 3, characterised in that the process is carried out in the presence of copper or copper compounds.

5. Process according to Claims 1 to 4, characterised in that an acid from the group comprising formic acid, acetic acid, trifluoroacetic acid, phosphoric acid, hydrochloric acid, sulphuric acid, perchloric acid and acid ion exchangers is added.

6. Process according to Claims 1 to 5, characterised in that the process is carried out at 50 to 190 °C.

7. Process according to Claims 1 to 6, characterised in that the process is carried out in the presence of a solvent.

8. Process according to Claims 1 to 7, characterised in that, relative to 2-butene-1,4-diol, 0.1 to 25 % by weight of catalytically active substances is used.

9. Process according to Claims 1 to 8, characterised in that, relative to 2-butene-1,4-diol, 0.5 to 40 % by weight of acid is added.

**Revendications**

1. Procédé de production de 1-butène-3,4-diol, caractérisé en ce qu'on traite thermiquement du 2-butène-1,4-diol en présence de substances à effet catalytique et avec addition d'un acide organique ou inorganique.

2. Procédé suivant la revendication 1, caractérisé en ce qu'il est mis en œuvre en présence d'éléments du troisième et du quatrième groupe principal du Système Périodique des éléments selon Mendelejew, de métaux de transition ou de composés de ces éléments ou de composés des métaux de transition.

3. Procédé suivant les revendications 1 et 2, caractérisé en ce qu'il est mis en œuvre en présence d'éléments des premier ou deuxième sous-groupes du Système Périodique des éléments selon Mendelejew ou de leurs composés.

4. Procédé suivant les revendications 1 à 3, caractérisé en ce qu'il est mis en œuvre en présence de cuivre ou de composés de cuivre.

5. Procédé suivant les revendications 1 à 4, caractérisé en ce qu'on ajoute un acide du groupe de l'acide formique, de l'acide acétique, de l'acide trifluoracétique, de l'acide phosphorique, de l'acide chlorhydrique, de l'acide sulfurique, de l'acide perchlorique et d'échangeurs d'ions acides.

6. Procédé suivant les revendications 1 à 5, caractérisé en ce qu'il est mis en œuvre à une température de 50 à 190 °C.

7. Procédé suivant les revendications 1 à 6, caractérisé en ce qu'il est mis en œuvre en présence d'un solvant.

8. Procédé suivant les revendications 1 à 7, caractérisé en ce qu'on utilise 0,1 à 25 % en poids de substances catalytiquement actives par rapport au 2-butène-1,4-diol.

9. Procédé suivant les revendications 1 à 8, caractérisé en ce qu'on ajoute 0,5 à 40 % en poids d'acide par rapport au 2-butène-1,4-diol.